# EUROPEAN PATENT SPECIFICATION

(11) **EP 4 593 777 B1**
(45) Date of publication and mention of the grant of the patent: **09.12.2026**
(21) Application number: 22798237.8
(22) Date of filing: 24.10.2022
(51) Int. Cl.: A61F 13/494, A61F 13/495, A61F 13/537, A61F 13/49, A61F 13/496

(54) **ABSORBENT BODY WITH COMPOSITE ISOLATION SHEET**
SAUGFÄHIGER KÖRPER MIT VERBUNDISOLIERUNGSFOLIE
CORPS ABSORBANT AVEC FEUILLE D'ISOLATION COMPOSITE

(30) Priority: 28.09.2022 WO PCT/CN2022/122097
(43) Date of publication of application: 06.08.2025
(73) Proprietor: The Procter & Gamble Company, Cincinnati, OH 45202 (US)
(72) Inventor: YAMAMOTO, Yoichiro, Guangzhou, Guangdong 510555 (CN); YE, Fengchun, Beijing 101312 (CN); FUJIKAWA, Hiroshi, Akashi, Hyogo 6740093 (JP); HUANG, Jiale, Guangzhou, Guangdong 510555 (CN); YUAN, Yi, Beijing 101312 (CN); WANG, Xinjun, Guangzhou, Guangdong 510555 (CN)
(74) Representative: P&G Patent Germany
(86) International application number: PCT/CN2022/126978
(87) International publication number: WO 2024/065908

(56) References cited:
- WO-A2-94/28844
- GB-A- 2 287 888
- US-A- 4 662 877

## Description

### FIELD OF INVENTION

This invention relates to absorbent bodies useful for absorbent articles such as diapers having a composite isolation sheet for isolating body exudates from the wearer's skin.

### BACKGROUND OF THE INVENTION

Absorbent articles for personal hygiene, such as disposable diapers, disposable pants, and adult incontinence undergarments, are designed to absorb and contain various body exudates, including urine, menses, low viscosity fecal matter, and solid fecal matter. Fecal material is often difficult to remove from the skin of the user, in particular from sensitive skin such as of young babies and such as the skin around the genitals. Moreover, it is known that when fecal material and urine meet on the skin, this may cause irritation and redness of the skin and sometimes even dermatitis of the skin.

One of the solutions to reduce the fecal material on the skin is to provide a means to isolate the fecal material immediately after discharge, away from the skin. For example, diapers having an elasticized sheet closest to the wearer with an opening, through which the feces can pass to a void space between the sheet closest to the wearer and the remainder of the absorbent body, have been developed. The fecal material is then stored underneath the sheet closest to the wearer, away from the skin.

Absorbent articles with means to isolate the fecal material have been known in the art, such as in EP357,298A and JP3,130,365B. US4,662,877 discloses a diaper that comprises a backing sheet shaped to have cut-away intermediate side portions to provide a narrowed crotch region, a narrow rectangular absorbent batt, a moisture permeable non-woven fibrous batt cover sheet and a hydrophobic substantially liquid impermeable non-woven fibrous facing sheet. A similar disclosure to that is found in GB2,287,888. WO94/28844 discloses a diaper having a first front waist region, a second rear waist region, a crotch region disposed between the front and rear waist regions, and a periphery which is defined by longitudinal edges and front and rear end edges. The diaper additionally has a lateral centerline and a longitudinal centerline.

While such absorbent articles have been known, they have shortcomings that have prevented significant commercial success. For example, those known in the art may be difficult to produce in today's high speed manufacturing process, as requiring elasticity along the shaped opening, or in various directions deviating from the machine direction.

Based on the foregoing, there is a need for an absorbent article having a composite isolation sheet for isolating body exudates from the wearer's skin while maintaining the performance of containment and wear comfort. There is also a need for such an absorbent article which may be economically manufactured.

### SUMMARY OF THE INVENTION

The present invention is directed to an absorbent body having a transverse direction, a longitudinal direction, a front end, a back end, a front region, a garment facing direction, and a wearer facing direction, comprising:
1) a water permeable topsheet;
2) a water impermeable backsheet;
3) an absorbent core disposed between the topsheet and the backsheet;
4) a composite isolation sheet bonded to the wearer facing side of the topsheet, the composite isolation sheet comprising:
   a) a pair of inner cuffs having a pair of side edges which are elasticized in the longitudinal direction;
   b) a front portion in the front region having a front longitudinal end point and a pair of front opening edges wherein the pair of front opening edges are not elasticized wherein the front portion has a longitudinal dimension F1 of from about 8% to about 50% of the longitudinal dimension of the absorbent body; and
   c) a front longitudinal elastic element disposed on the front portion, wherein the front longitudinal elastic element extends substantially in the longitudinal direction, characterized in that the front longitudinal elastic element is an elastic body extending along the longitudinal axis between the front longitudinal end point towards the front end.

The present invention is also directed to an absorbent article comprising said absorbent body.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings and which like designations are used to designate substantially identical elements, and in which:
Figures 1A-1B are schematic plan views of embodiments of absorbent bodies and absorbent articles showing the wearer facing side, and with any elastic elements fully stretched, wherein Figure 1B represents an embodiment of the absorbent body and the absorbent article according to the invention.
Figure 2A is a transverse schematic cross section view taken along line A-A of Figures 1A-1B with the thickness exaggerated, and elastic elements contracted.
Figure 2B is a transverse schematic cross section view taken along line B-B of Figure 1A with the thickness exaggerated, and elastic elements contracted.
Figures 3A-3F are schematic cross section views of embodiments of the composite isolating sheet disclosed herein.

### DEFINITIONS

As used herein, the following terms shall have the meaning specified thereafter:
"Absorbent article" refers to articles of wear which may be in the form of taped type diapers, pant type diapers, incontinent briefs, feminine hygiene garments, and the like. The "absorbent article" may be so configured to absorb and contain various exudates such as urine, feces, and menses discharged from the body. The "absorbent article" may refer to a combined merchandise of an outer cover adaptable to be joined with a separable disposable absorbent insert for providing absorbent and containment function, such as those disclosed in WO2011/087503A.

"Longitudinal" refers to a direction running substantially perpendicular from a waist edge to an opposing waist edge of the article and generally parallel to the maximum linear dimension of the article.

"Transverse" refers to a direction perpendicular to the longitudinal direction.

"Proximal" and "distal" refer respectively to the position closer or farther relative to the longitudinal center of the article.

"Wearer-facing" and "garment-facing" refer respectively to the relative location of an element or a surface of an element or group of elements. "Wearer-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).

"Disposed" refers to an element being located in a particular place or position.

"Joined" refers to configurations whereby an element is directly secured to another element by affixing the element directly to the other element and to configurations whereby an element is indirectly secured to another element by affixing the element to intermediate member(s) which in turn are affixed to the other element.

"Film" refers to a sheet-like material wherein the length and width of the material far exceed the thickness of the material. Typically, films have a thickness of about 0.5 mm or less.

"Nonwoven", nonwoven layer" or "nonwoven web" are used interchangeably to mean an engineered fibrous assembly, primarily planar, which has been given a designed level of structural integrity by physical and/or chemical means, excluding weaving, knitting or papermaking (ISO 9092:2019 definition). The directionally or randomly orientated fibers, are bonded by friction, and/or cohesion and/or adhesion. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m² or gsm).

"Water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, i.e., may be "vapor-permeable".

"Hydrophilic" describes surfaces of substrates which are wettable by aqueous fluids (e.g., aqueous body fluids) deposited on these substrates. Hydrophilicity and wettability are typically defined in terms of contact angle and the strike-through time of the fluids, for example through a nonwoven fabric. This is discussed in detail in the American Chemical Society publication entitled "Contact Angle, Wettability and Adhesion", edited by Robert F. Gould (Copyright 1964). A surface of a substrate is said to be wetted by a fluid (i.e., hydrophilic) when either the contact angle between the fluid and the surface is less than 90°, or when the fluid tends to spread spontaneously across the surface of the substrate, both conditions are normally co-existing. Conversely, a substrate is considered to be "hydrophobic" if the contact angle is greater than 90° and the fluid does not spread spontaneously across the surface of the fiber.

"Extendibility" and "extensible" mean that the width or length of the component in a relaxed state can be extended or increased.

"Elasticated" and "elasticized" mean that a component comprises at least a portion made of elastic material.

"Elongatable material", "extensible material", or "stretchable material" are used interchangeably and refer to a material that, upon application of a biasing force, can stretch to an elongated length of at least about 110% of its relaxed, original length (i.e. can stretch to 10 percent more than its original length), without rupture or breakage, and upon release of the applied force, shows little recovery, less than about 20% of its elongation without complete rupture or breakage as measured by EDANA method 20.2-89. In the event such an elongatable material recovers at least 40% of its elongation upon release of the applied force, the elongatable material will be considered to be "elastic" or "elastomeric." For example, an elastic material that has an initial length of 100mm can extend at least to 150mm, and upon removal of the force retracts to a length of at least 130mm (i.e., exhibiting a 40% recovery). In the event the material recovers less than 40% of its elongation upon release of the applied force, the elongatable material will be considered to be "substantially non-elastic" or "substantially non-elastomeric". For example, an elongatable material that has an initial length of 100mm can extend at least to 150mm, and upon removal of the force retracts to a length of at least 145mm (i.e., exhibiting a 10% recovery).

"Dimension", "Length", "Width", "Pitch", "Diameter", "Aspect Ratio", "Angle", and "Area" of the article are all measured in a state wherein the article is extended to the Full Stretch Circumference W1 according to the "Whole Article Force Measurement" herein, and utilizing a ruler or a loupe, unless specified otherwise.

"Artwork" refers to a visual presentation to the naked eye, which is provided by printing or otherwise, and having a color. Printing includes various methods and apparatus well known to those skilled in the art such as lithographic, screen printing, flexographic, and gravure ink jet printing techniques.

"Color" or "Colored" as referred to herein includes any primary color except color white, i.e., black, red, blue, violet, orange, yellow, green, and indigo as well as any declination thereof or mixture thereof. The color white is defined as those colors having a L* value of at least 94, an a* value equal to 0 ± 2, and a b* value equal to 0 ± 2 according to the CIE L* a* b* color system.

### DETAILED DESCRIPTION OF THE INVENTION

### Absorbent Body and Absorbent Article

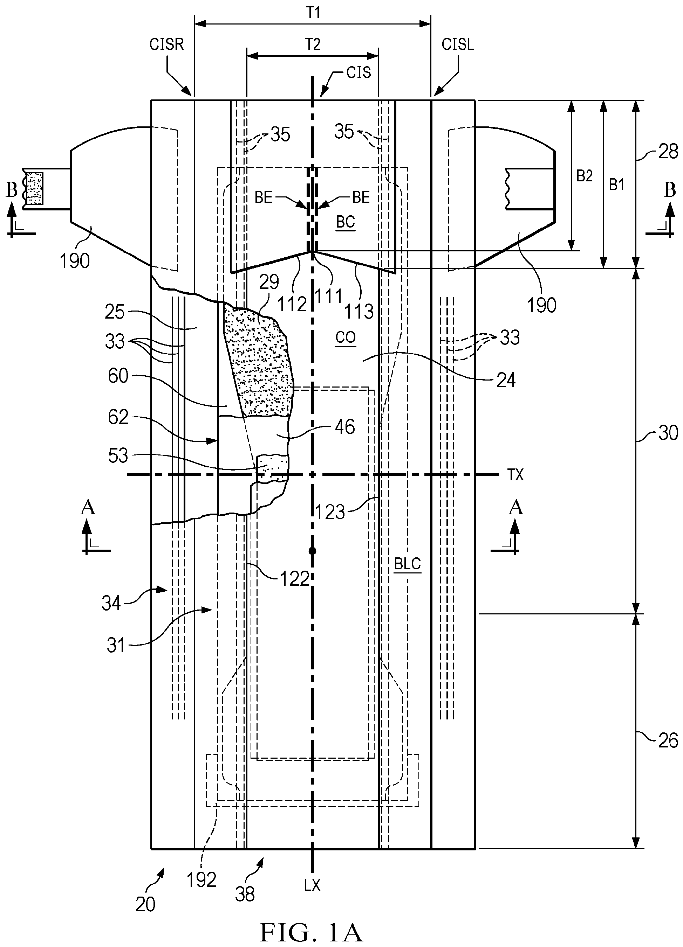

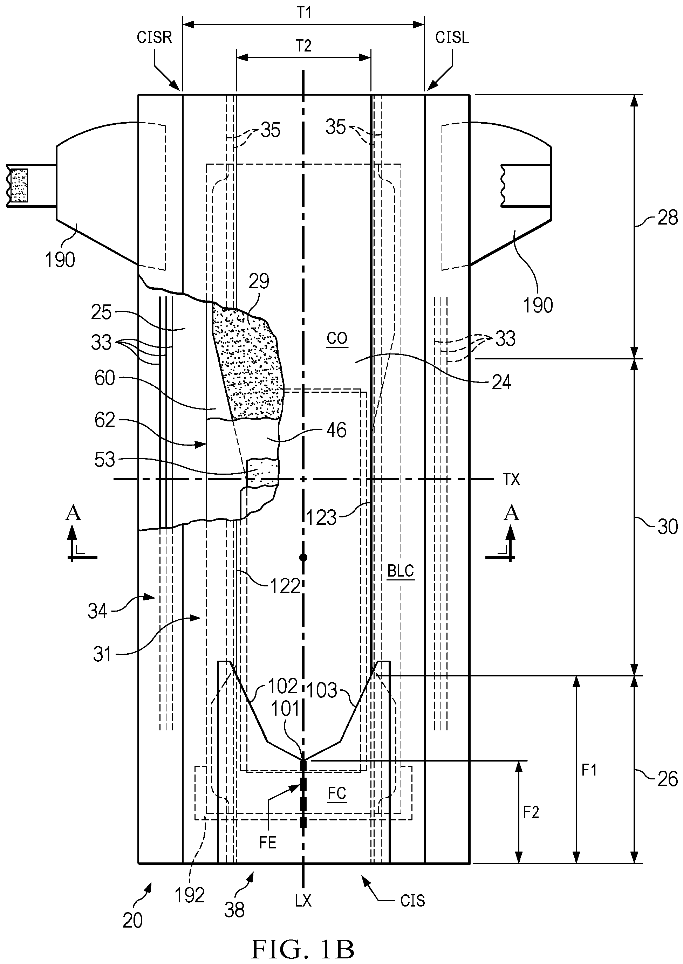

Figures 1A and 1B are schematic plan views of an absorbent article 20 of the taped type carrying the absorbent body 38, wherein Figure 1B is a schematic plan view of the absorbent article 20 of the tape type carrying the absorbent body 38 of the present invention. The absorbent article 20 and absorbent body 38 have a longitudinal centerline LX which also serves as the longitudinal axis, and a transverse centerline TX which also serves as the transverse axis, a front end and a back end. The absorbent body 38 has a wearer-facing surface, a garment facing surface, a liquid permeable topsheet, a liquid impermeable backsheet, an absorbent core disposed between the topsheet and the backsheet, and a composite isolation sheet bonded to the wearer facing side of the topsheet. The absorbent body 38 of the present invention may be assembled together with an application means, wherein the application means is selected from the group of a fastening means and an elastic belt. The exemplary absorbent articles of Figures 1A and 1B comprise a fastening means comprising a pair of elongate members 190 and a receiving member 190, the elongate members 190 transversely protruding from the left and right side edges of the back region and fastenable with the receiving member 190 disposed on the front region. While not shown, the application means may also be an elastic belt, and the absorbent article 20 may take the form of a pant.

### Topsheet and Backsheet

The absorbent body 38 of the present invention comprises a water permeable topsheet 24 that may be positioned at least in partial contact or close proximity to a wearer. Suitable topsheets 24 may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. The topsheet 24 is generally supple, soft feeling, and non-irritating to a wearer's skin. The topsheet 24 is liquid permeable, permitting bodily fluids to readily penetrate through the thickness of the topsheet 24. One topsheet useful herein is available from Fibertex NiLai, Malaysia with tradename H30501221 or FQN Hazlet NJ with tradename SB1206169. Any portion of the topsheet 24 may be coated with a lotion or skin care composition as is known in the art. Examples of suitable lotions include those described in US5,607,760; US5,609,587; US5,635,191; and US5,643,588.

The absorbent body 38 of the present invention comprises a water impermeable backsheet 25 which is designed to prevent the exudates absorbed by and contained within the absorbent core (59) from soiling articles that may contact the absorbent article, such as bed sheets and undergarments. The backsheet 25 may be positioned such that it extends beyond the absorbent core 62 in both the longitudinal direction and the transverse direction. Suitable backsheet 25 materials include films such as those manufactured by Plaster Argentina with tradename PLBA NBBS 10-12GSM PR V1. Other suitable backsheet 25 materials may include breathable materials that permit vapors to escape from the absorbent article while still preventing exudates from passing through the backsheet 25. Exemplary breathable materials may include materials such as woven webs, nonwoven webs, composite materials such as film-coated nonwoven webs, and microporous films such as manufactured by Daika Japan with tradename MPF DKH-180 15G V7 and manufactured by Berry Nashville, TN with trademark BR-137P V13. Such breathable composite materials are described in greater detail in WO 95/16746 and US5,865,823. Other breathable backsheets including nonwoven webs and apertured formed films are described in US5,571,096. An exemplary, suitable backsheet is disclosed in US6,107,537. Other suitable materials and/or manufacturing techniques may be used to provide a suitable backsheet including, but not limited to, surface treatments, particular film selections and processing, particular filament selections and processing, etc.

### Absorbent Core

Referring to Figure 1A, the absorbent body 38 comprises an absorbent core 62 for absorbing and containing body exudates disposed on the wearer facing side. The absorbent core 62 may include an absorbent layer and an acquisition system 51. The absorbent layer is the region wherein absorbent materials 29 having a high retention capacity, such as superabsorbent polymers, are present. The absorbent layer may be substantially cellulose free. Alternatively, the absorbent layer may contain cellulose. There may be an absorbent layer mainly comprising cellulose, and another absorbent layer mainly comprising superabsorbent polymers.

Superabsorbent polymers of the absorbent layer may be disposed between first and second layers of material immobilized by a fibrous layer of thermoplastic adhesive material. The first and second layers of materials may be nonwoven fibrous webs including synthetic fibers, such as mono-constituent fibers of PE, PET and PP, multi constituent fibers such as side by side, core/sheath or island in the sea type fibers. Such synthetic fibers may be formed via a spunbonding process or a meltblowing process.

The absorbent body 38 may also contain an acquisition system 51 for facilitating the acquisition and the distribution of body exudates, and may be placed between the topsheet 24 and the absorbent layer. The acquisition system 51 may include cellulosic fibers. The absorbent layers may be disposed in plurality in the absorbent core 62. Some portions of the absorbent layers may be configured to have substantially no absorbent material to form a channel or a plurality of channels. Channels may be useful for allowing the absorbent core 62 to bend upon swelling with fluids, such that the center region conforms to the wearer's body after swelling and prevent sagging of the article. The channels may also be formed in the acquisition system 51, and may be configured to at least partly match the channels of the absorbent layer in the thickness direction. The acquisition system 51 may comprise a liquid management layer (53) directly under the topsheet. The function of such a layer is to rapidly acquire the fluid from the topsheet 24 away from the wearer-facing side and/or to distribute over a larger area so it is more efficiently absorbed by the absorbent core. It is also possible that such a liquid management layer 53 may be placed between the backsheet 25 and the absorbent core. The liquid management layer may be a spunlace nonwoven comprising viscose, PET, CoPET/PET fibers, and combinations thereof.

The absorbent core 62 may comprise a high loft material encompassing superabsorbent polymers. The term "high loft" refers to low density bulky fabrics, as compared to flat, paper-like fabrics. High loft webs are characterized by a relatively high porosity. This means that there is a relatively high amount of void space in which superabsorbent polymer particles can be distributed. The high loft material (without the superabsorbent particles) of the invention may have a density at a pressure of 4.14kPa (0.6 psi) below 0.20 g/cm³, in particular ranging from 0.05 g/cm³ to 0.15 g/cm³. The high loft layer (without the superabsorbent particles) may have a density at a pressure of 2.07 kPa (0.3 psi) below 0.20 g/cm³, in particular ranging from 0.02 g/cm³ to 0.15 g/cm³. The high loft layer (without the superabsorbent particles) of the invention may have a density at a pressure of 0.83 kPa (0.12 psi) below 0.15 g/cm³, in particular ranging from 0.01 g/cm³ to 0.15 g/cm³, and a basis weight of from 15 to 500 gsm, preferably 30~200 gsm, such as those described in US 2021/0361497 Al. The absorbent core 62 comprising high loft material encompassing superabsorbent polymers may also contain channels.

Alternatively, the absorbent core 62 may comprise an absorbent layer having superabsorbent polymers disposed between first and second layers of nonwoven material immobilized by a fibrous layer of thermoplastic adhesive material (not shown). The first and second layers of nonwoven materials may be relatively low basis weight nonwoven fibrous webs including synthetic fibers, such as mono-constituent fibers of PE, PET and PP, multi constituent fibers such as side by side, core/sheath or island in the sea type fibers. Such synthetic fibers may be formed via a spunbonding process or a meltblowing process. In such embodiments, a) the intermediate layer 60 may be hydrophobic and the lower substrate layer 46 may be hydrophilic; or b) the intermediate layer 60 and the lower substrate layer 46 may both be hydrophilic and the intermediate layer 60 may be less hydrophilic than the lower substrate layer 46; or c) the intermediate layer 60 and the lower substrate layer 46 may both be hydrophobic and the lower substrate layer 46 may be less hydrophobic than the intermediate layer 60.

### Composite Isolation Sheet

Referring to Figures 1A-1B, the absorbent body 38 comprises a composite isolation sheet (CIS) bonded to the wearer facing side of the topsheet for isolating body exudates from the wearer's skin. The composite isolation sheet (CIS) comprises a) a pair of inner cuffs (BLC) having a pair of side edges which are elasticized in the longitudinal direction; and b) either a front portion (FC) or a back portion (BC). A longitudinal elastic element is disposed on the front portion (FC) or the back portion (BC), wherein the longitudinal elastic element extends substantially in the longitudinal direction. The region and area of the composite isolation sheet (CIS) is defined by the front end of the absorbent body, the back end of the absorbent body, a left side end (CISL), and a right side end (CISR), the left and right side ends (CISL, CISR) being bonded to the topsheet 24. Although the substrate for providing the composite isolation sheet (CIS) may extend into other elements of the absorbent body 38 such as the outer cuffs as described later, such other elements are not considered the composite isolation sheet (CIS).

Still referring to Figures 1A-1B, the composite isolation sheet (CIS) comprises a pair of inner cuffs (BLC) extending for the remainder of the longitudinal length of the absorbent body except for the front portion (FC) or the back portion (BC), and forms a pair of side edges which are elasticized in the longitudinal direction along the longitudinally extending side edges 122, 123. The pair of side edges 122, 123 are elasticized at least in the center region 30 as defined later, and may be devoid of elasticity adjacent the front end and/or back end of the absorbent body. Referring to Figure 1A, when the composite isolation sheet (CIS) comprises a back portion (BC), the crossing point with the back portion (BC) of the side edges 122, 123 may be in active elasticity. Referring to Figure 1B, according to the invention the composite isolation sheet (CIS) comprises a front portion (FC), the crossing point with the front portion (FC) of the side edges 122, 123 may be in active elasticity. The pair of side edges 122, 123 of the inner cuffs (BLC) are elasticized by longitudinally extending elastic bodies (35) disposed adjacent the side edges 122, 123. The elastic bodies for elasticizing the side edges 122, 123 may be provided in a plurality of elastic strands (35). The inner cuff (BLC) may have a transverse dimension (T1) between the left and right side ends (CISL, CISR) of from about 120mm to about 170mm. Such transverse dimension may be the same for the entire composite isolation sheet (CIS). The transverse distance (T2) between the side edges 122, 123, when the inner cuffs (BLC) are completely laid flat on the topsheet, may be from about 60mm to about 140mm.

Referring to Figure 1A, when the composite isolation sheet (CIS) comprises a back portion (BC), the back region 28 is defined by the line extending parallel to the transverse axis matching the crossing point of the back portion (BC) and the pair of inner cuffs (BLC), while the front region 26 is defined by a line extending parallel to the transverse axis at one third of the entire longitudinal length (L1) of the absorbent body from the front end. The center region 30 is defined between the front region 26 and the back region (28). When the composite isolation sheet (CIS) comprises a back portion (BC), the composite isolation sheet (CIS) upon wear does not cover the anal orifice of the wearer. Still referring to Figure 1A, when the composite isolation sheet (CIS) comprises a back portion (BC), the back portion (BC) has a back longitudinal end point 111 and a pair of back opening edges (112, 113), wherein the back opening edges (112, 113) are so configured such that the back longitudinal end point 111 is positioned on the longitudinal axis and within the back region (28). The back opening edges (112, 113) may be curved, straight, or formed by a series of straight lines. The back opening edges (112, 113) are not elasticized. The back portion (BC) has a longitudinal dimension (B1) which may be from about 5% to about 35% of the longitudinal dimension of the absorbent body (L1).

Still referring to Figure 1A, when the composite isolation sheet (CIS) comprises a back portion (BC), a back longitudinal elastic element (BE) is disposed on the back portion, wherein the back longitudinal elastic element extends substantially in the longitudinal direction. The back longitudinal elastic body (BE) may extend from the back longitudinal end point 111 towards the back end. The distance from the back longitudinal end point 111 to the back end, when the back longitudinal elastic body is fully stretched, is represented by B2. The back longitudinal elastic body (BE) may extend for only a portion of B2. While not shown, the back longitudinal elastic body (BE) may extend for the entirety of B2, or in positions closer to the back end, or in the middle of the line extending between the back longitudinal end point 111 and the back end. In the back portion (BC), B1 may equal B2. The distance from the back longitudinal end point 111 to the back end, when fully contracted by the back longitudinal elastic body (BE), is represented by B3. The force provided by the back longitudinal elastic body (BE) may be such so that B3 is from about 30% to about 85% of B2.

Referring to Figure 1B, according to the invention the composite isolation sheet (CIS) comprises a front portion (FC), the front region 26 is defined by the line extending parallel to the transverse axis matching the crossing point of the front portion (FC) and the pair of cuffs (BLC), while the back region 28 is defined by a line extending parallel to the transverse axis at one third of the entire longitudinal length (L1) of the absorbent body from the back end. The center region 30 is defined between the front region 26 and the back region (28). When the composite isolation sheet (CIS) comprises a front portion (FC), the composite isolation sheet (CIS) upon wear does not cover the urethral orifice of the wearer. Still referring to Figure 1B, the composite isolation sheet (CIS) comprises a front portion (FC) having a front longitudinal end point 101 and a pair of front opening edges 102, 103, wherein the front opening edges 102, 103 are so configured such that the front longitudinal end point 101 is positioned on the longitudinal axis and within the front region 26. The front opening edges 102, 103 may be curved, straight, or formed by a series of straight lines. The front opening edges 102, 103 are not elasticized. The front portion (FC) has a longitudinal dimension (F1) which may be from about 8% to about 50%, or from about 22% to about 50% of the longitudinal dimension (L1) of the absorbent body 38.

Still referring to Figure 1B, the composite isolation sheet (CIS) comprises a front portion (FC), a front longitudinal elastic element (FE) is disposed on the front portion, wherein the front longitudinal elastic element extends substantially in the longitudinal direction. The front longitudinal elastic body (FE) extends from the front longitudinal end point 101 towards the front end. The distance from the front longitudinal end point 101 to the front end, when the front longitudinal elastic body is fully stretched, is represented by F2. The front longitudinal elastic body (FE) may extend for only a portion of F2. While not shown, the longitudinal elastic body (FE) may extend for the entirety of F2, or in positions closer to the front end, or in the middle of the line extending between the front longitudinal end point 101 and the front end. The distance from the front longitudinal end point 101 to the front end, when fully contracted by the front longitudinal elastic body (FE), is represented by F3. The force provided by the front longitudinal elastic body (FE) may be such so that F3 is from about 30% to about 85% of F2.

Whether disposed on the front portion or on the back portion, the longitudinal elastic element (FE, BE) may be provided in a single, or a plurality of longitudinal elastic body/bodies. The longitudinal elastic body may be an elastic ribbon or an elastic strand. When the longitudinal elastic element is a single elastic ribbon or strand, the longitudinal elastic body (FE, BE) may be disposed on and extending along the longitudinal axis (LX) between the front longitudinal end point 101 towards the front end, or the back longitudinal end point 111 towards the back end. As in Figure 1B, the longitudinal elastic body (FE) is disposed on the longitudinal axis (LX). As in Figure 1A, the longitudinal elastic body (BE) may be in a pair or any other even number and disposed in a line symmetric position about the longitudinal axis (LX) on the left and right portions of the back portion (BC). While not shown, the longitudinal elastic element may be in 3 bodies and disposed along the longitudinal axis (LX) as well as in a symmetric position against the longitudinal axis (LX) on the left and right portions of the front portion (FC) or the back portion (BC).

The longitudinal elastic body may be elastic strands having a thickness of about 1100dtex or less, wherein at least 2 of the elastic strands are disposed in an array in close proximity to each other in the transverse direction. Within the array, the transverse distance between the elastic strands may be in a dimension of at least about 2mm to no more than about 4mm. There may be 2, 3, or 4 elastic strands in one array. Without being bound by theory, by disposing the elastic stands in such close proximity to each other, the array exhibits a behavior as if it were one elastic body with a certain width, and provides various benefits. For example, the array provides more or less the combined tensile force of the elastic stands in the array, such that each elastic strand may be disposed at a much lower tensile force. Further, fine gathers may be created in the interval within the array. Such fine gathers combined with the elastic strands of the array, apply a tensile force to the wearer over a distributed width, compared to the width of a single elastic strand. Namely, the array exhibits a behavior as if it were one elastic body. As such, the array may provide a soft fit and may also reduce red marking on the skin contacted by the front or back portion (FC, BC).

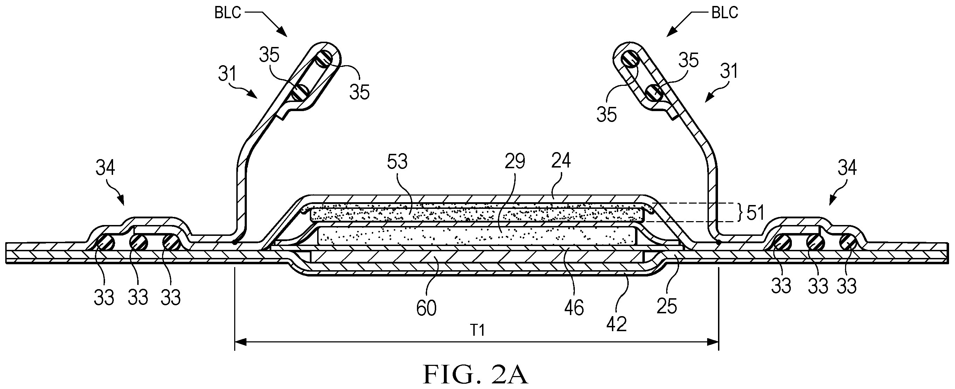

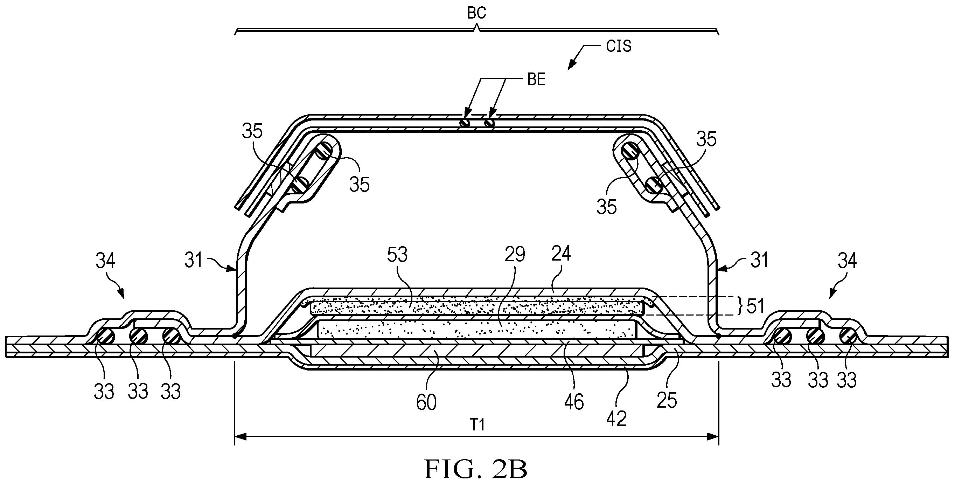

Accordingly, in that the composite isolation sheet (CIS) holds tension provided by the combination of elastic bodies (35) of the side edges and the longitudinal elastic element (FE, BE) when contracted upon wear, the composite isolation sheet (CIS) is raised from the topsheet 24. The dimensions of the composite isolation sheet (CIS) and the tension provided by the elastic bodies (35) of the side edges and the longitudinal elastic element are adjusted so that, upon wear, the front portion (FC) or the back portion (BC) does not cover the anal or urethral orifice of the wearer. Referring to Figure 2A representing the schematic cross section of Figure 1A-1B taken along line A-A in the center region 30, only the elasticized side edges 122, 123 are observed. Referring to Figure 2B representing the schematic cross section of Figure 1A taken along line B-B in the back portion (BC), the back portion and back longitudinal elastic body (BE) are also observed. The composite isolation sheet (CIS) of the present invention is devoid of elasticity along the pair of front opening edges 102, 103 as well as the pair of back opening edges 112, 113. Even though there is such absence of elasticity around certain portions of the composite isolation sheet (CIS), there may be provided sufficient tension by the combination of the elasticized side edges 122, 123 and the longitudinal elastic element. Such configuration of the composite isolation sheet (CIS) enables providing elasticization generally parallel to the longitudinal axis (LX) of the absorbent body. Such configuration of the composite isolation sheet (CIS) of the present invention is advantageous in that, when the machine direction for manufacturing the absorbent body matches the longitudinal direction, any elastic body to be disposed on the composite isolation sheet (CIS) may also be advanced in the machine direction for attachment.

Material for making the composite isolation sheet (CIS) may be made from a substantially liquid impervious material. The material may be an SMS nonwoven or an SMMS nonwoven material, or a nonwoven component layer comprising fine fibers having an average diameter of less than 1 micron. One useful combination of nonwoven fabric webs may include spunbond, meltblown, spunbond ("SMS") webs comprising outer layers of spunbond thermoplastics (e.g., polyolefins) and an interior layer of meltblown thermoplastics. Suitable composite isolation sheet material useful herein include those of SMS type available from Toray Polytech Nantong China with tradename LIVSEN SMS 13, available from FQN Hazlet NJ with tradename SM15009270, and available from Fibertex Aalborg Denmark with tradename B10160HS. The material may be treated, by region or in part of a region, with a lotion or a hydrophobic surface coating to provide various physical properties. Material for making the composite isolation sheet (CIS) may have a hydrostatic head of greater than about 2mbar, or greater than about 3mbar, or greater than about 4mbar. The material may have a hydrostatic head of less than about 200mbar, or less than about 100mbar, or less than about 75mbar, or less than about 50mbar, or less than about 25mbar, or less than about 15mbar. The material may have an opacity of from about 15% to about 50% hunter opacity, or from about 20% to about 45% hunter opacity. The material may have an opacity of from about 45% to about 75% hunter opacity; or from about 50% to about 70% hunter opacity. The material may have an air permeability of less than about 50 m³/m²/min; or less than about 45 m³/m²/min. The material may have an air permeability of greater than about 5 m³/m²/min; or greater than about 10 m³/m²/min; or greater than about 15 m³/m²/min; or greater than about 20 m³/m²/min.

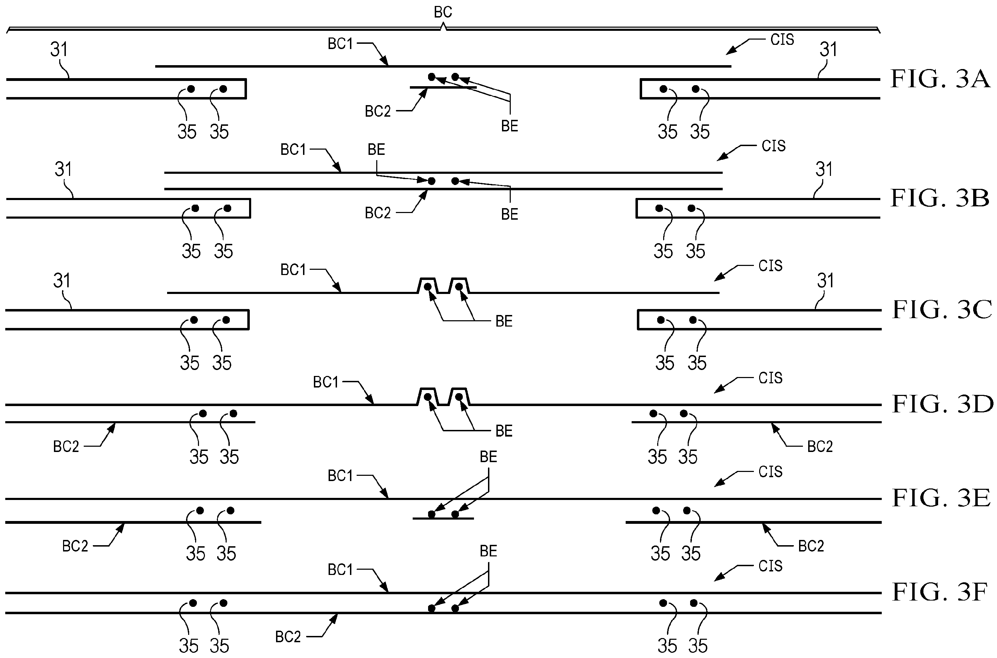

Referring to Figures 3A - 3F, various configurations for forming the composite isolation sheet (CIS) are disclosed. Figures 3A - 3F are schematic cross section views taken at line B-B of Figure 1A, with the substrates for making the composite isolating sheet (CIS) extended laterally and detached from the remainder of the absorbent body 38.

Figures 3A - 3C are related to configurations wherein the inner cuff (BLC) is formed by a different part from the back portion (BC). In these configurations, the inner cuff (BLC) is formed by an inner cuff part (31) extending the entire length of the absorbent body 38. When taking such configuration for the inner cuff (BLC), at least the center region 30 is in active elasticity. When taking such configuration for the inner cuff (BLC), the inner cuff part (31) overlapping the back portion (BC) may be devoid of active elasticity, particularly along the back end of the absorbent core 38. Still referring to Figures 3A - 3C, the back portion (BC) is formed by a back part that is bonded on the wearer-facing side of the inner cuff part (31).

Referring to Figure 3A, the back longitudinal elastic bodies (BE) may be disposed between an outer back portion sheet (BC1) and an inner back portion sheet (BC2). The inner back portion sheet (BC2) may have a shorter lateral dimension than the outer back portion sheet (BC1) as in Figure 3A. Referring to Figure 3C, the back longitudinal elastic bodies (BE) may be wrapped in a single layer of material. Such configurations of Figures 3A or 3C may provide the back portion (BC) thin and breathable. Referring to Figures 2B and 3B, the inner back portion sheet (BC2) may have the same lateral dimension as the outer back portion sheet (BC1). Such a configuration enables secure attachment of the back longitudinal elastic bodies (BE), and further provides improved protection in this area.

Figures 3D - 3F are related to configurations wherein the inner cuff (BLC) and the back portion (BC) are formed by the same integral part. In these configurations, the elasticizing of the side edges of the inner cuff (BLC) and the back longitudinal elastic bodies (BE) may be provided by the outer back portion sheet (BC1) and the inner back portion sheet (BC2). Referring to Figure 3D, the back longitudinal elastic bodies (BE) may be wrapped in a single layer of material. Referring to Figures 3E and 3F, the elasticizing of the side edges of the inner cuff (BLC) and the back longitudinal elastic bodies (BE) may be provided between the outer back portion sheet (BC1) and the inner back portion sheet (BC2).

For any of the configurations of Figures 3A - 3F, the front portion (FC) may be formed in the same way as the back portion (BC) as described above.

### Other components

Referring to Figures 1A and 2A, the absorbent body 38 of the present invention may further comprise components that improve leakage prevention, wearability, fit, or aesthetic aspects of the resulting absorbent article.

The absorbent body 38 may comprise gasketing leg cuffs 34. The gasketing leg cuffs (34) may be at least partially enclosed between the topsheet 24 and the backsheet 25, and may be placed transversely outward relative to the composite isolation sheet (CIS). The gasketing leg cuffs (34) may provide sealing around the thighs of the wearer. Usually each gasketing leg cuff (34) will comprise one or more elastic string or elastic element 33 comprised in the absorbent body 38 for example between the topsheet 24 and backsheet 25 in the area of the leg openings. The substrate for making the composite isolation sheet (CIS) may extend transversely and be utilized for forming the gasketing leg cuffs 34.

The absorbent body 38 of the present invention may comprise an outer cover 42 located on the garment-facing side of the backsheet 25. The outer cover 42 may be made of a soft, non-woven material. The outer cover 42 and the backsheet 25 may be joined together by adhesive or any other suitable material or method. A particularly suitable outer cover 42 is available from Fibertex NiLai Malaysia with tradename A10160EJ - MALAYSIA and available from FQN Hazlet NJ with tradename SM1104174.

The absorbent core 62 may comprise an intermediate layer 60 between the layer of absorbent material and the backsheet 25. The intermediate layer 60 may be in direct contact with the layer of absorbent material 29and with the backsheet 25. The intermediate layer 60 may be useful as a masking layer to isolate the superabsorbent polymer particles in the layer of absorbent material from the backsheet 25, thus reducing graininess feeling and improving the tactile properties of the garment-facing side of the article, especially for absorbent core 62 containing a high level of superabsorbent polymer particles. The intermediate layer 60 may also isolate the exudates which have been absorbed in the layer of absorbent material from the garment-facing side of the article, as this may be visually unpleasant to the caregiver. Thus by having an intermediate layer with a relatively high opacity, stains in the layer of absorbent material (e.g. from urine or feces) can be concealed from view, when looking at the backsheet 25 of the absorbent article during use. The hunter opacity in the dry state of the intermediate layer may be at least 25%, or at least 40%, or at least 50%, or at least 70%. The intermediate layer 60 can also help reduce the residual moisture in contact with the backsheet 25, which may lead to cold/wet feeling for the caregiver, or may lead to the wearer mistaking the cold/wet feeling as liquid leaking out of the absorbent article. The intermediate layer 60 may also serve as a temporary reservoir for liquid that had not been absorbed fast enough by the layer of absorbent material.

### Opacity Method

Opacity is measured using a 0° illumination/45° detection, circumferential optical geometry, spectrophotometer with a computer interface such as the HunterLab LabScan XE running Universal Software (available from Hunter Associates Laboratory Inc., Reston, VA) or equivalent instrument. Instrument calibration and measurements are made using the standard white and black calibration plates provided by the vendor. All testing is performed in a room maintained at 23 ± 2 °C and 50 ± 2 % relative humidity.

The spectrophotometer is configured for the XYZ color scale, D65 illuminant, 10 ° standard observers, with UV filter set to nominal. The instrument is standardized according to the manufacturer's procedures using the 0.7 inch port size and 0.5 inch area view. After calibration, the software is set to the Y opacity procedure which prompts the operator to cover the sample with either the white or black calibration tile during the measurement.

Articles are pre-conditioned at 23 °C ± 2 C° and 50% ± 2% relative humidity for two hours prior to testing. To obtain a specimen, the article is stretched flat on a bench, body facing surface upward, and the total longitudinal length of the article is measured. A testing site is selected at the longitudinal midpoint of the article or the part. Using scissors, a test specimen is cut in 60 mm square or as close as possible to 60mm. Any elastic members are removed.

The specimen is placed over the measurement port. The specimen should completely cover the port with the surface corresponding to the wearer-facing surface of the specimen directed toward the port. The specimen is gently extended until taut in its longitudinal direction so that the specimen lies flat against the port plate. Adhesive tape is applied to secure the specimen to the port plate in its extended state for testing. Tape should not cover any portion of the measurement port. The specimen is then covered with the white standard plate. A reading is taken, then the white tile is removed and replaced with the black standard tile without moving the specimen. A second reading is taken, and the opacity is calculated as follows:$Opacity = \left(Y value\left(black backing\right) / Y value\left(white backing\right)\right) \times 100$

Specimens from five identical articles are analyzed and their opacity results recorded. The average opacity is calculated and reported to the nearest 0.01%.

### Air Permeability Test

Air permeability is tested using a TexTest FX3300 Air Permeability Tester (available from Advanced Testing Instruments, Greer, SC) with a custom made 1 cm² circular aperture (also available from Advanced Testing Instruments) or equivalent instrument. The instrument is calibrated according to the manufacturer's procedures. All testing is performed in a room maintained at 23 °C ± 2 C° and 50 % ± 2 % relative humidity.

The articles are pre-conditioned at 23 °C ± 2 C° and 50% ± 2% relative humidity for two hours prior to testing. To obtain a specimen, the article is stretched flat on a bench, body facing surface upward, and the total longitudinal length of the article is measured. A testing site is selected at the longitudinal midpoint of the article or the part. Using scissors, a test specimen is cut in 30 mm square or as close as possible to 30 mm. Any elastic members are removed.

The specimen is centered over the measurement port. The specimen should completely cover the port with the surface corresponding to the wearer-facing surface of the specimen directed toward the port. The specimen is gently extended in its longitudinal direction until taut so that the specimen lies flat across the port. Adhesive tape is applied to secure the specimen across the port in its extended state for testing. Tape should not cover any portion of the measurement port. The test pressure is set to allow air to pass through the specimen. For non-woven specimen the pressure is set for 125 Pa and for specimen containing films 2125 Pa is used. The sample ring is closed and the measuring range is adjusted until the range indicator shows green to indicate that the measurement is within the accepted limits of the instrument. The air permeability is recorded to the nearest 0.1 m3/m2/min.

### Hydrostatic Head Test

Hydrostatic head is tested using a TexTest FX3000 Hydrostatic Head Tester (available from Advanced Testing Instruments, Greer, SC) with a custom made 1.5 cm2 circular measurement port (also available from Advanced Testing Instruments). Two annular sleeve rings, the same dimensions as the gaskets around the measurement ports, are cut from the standard protective sleeves for fine nonwovens (part FX3000-NWH, available from Advanced Testing Instruments). The sleeve rings are then adhered with two-sided adhesive tape to the sample facing surfaces of the upper and lower gaskets of the TexTest instrument to protect the specimen during clamping. Standardize the instrument according to the manufacturer's procedures. All testing is performed in a room maintained at about 23 °C ± 2 C° and about 50 % ± 2 % relative humidity.

Precondition the articles at about 23 °C ± 2 C° and about 50% ± 2% relative humidity for two hours prior to testing. To obtain a specimen, lay the article stretched flat on a bench, body facing surface upward, and measure the total longitudinal length of the article. A testing site is selected at the longitudinal midpoint of the article or the part. Using scissors, a test specimen is cut in 70 mm square or as close as possible to 70 mm. Any elastic members are removed.

Place the specimen centered over the port of the upper test head. The specimen should completely cover the port with the surface corresponding to the garment-facing surface of the specimen directed toward the port (wearer-facing surface will then be facing the water). Gently extend the specimen taut in its longitudinal direction so that the specimen lies flat against the upper test plate. Adhesive tape is applied to secure the specimen to the test plate in its extended state for testing. Tape should not cover any portion of the measurement port.

Fill the TexTest syringe with distilled water, adding the water through the measurement port of the lower test plate. The water level should be filled to the top of the lower gasket. Mount the upper test head onto the instrument and lower the test head to make a seal around the specimen. The test speed is set to 3 mbar/min for samples that have a hydrostatic head of 50 mbar or less and a speed of 60 mbar/min for samples with a hydrostatic head above 50 mbar. Start the test and observe the specimen surface to detect water droplets penetrating the surface. The test is terminated when one drop is detected on the surface of the specimen or the pressure exceeds 200 mbar. Record the pressure to the nearest 0.5 mbar or record as >200 mbar if there was no penetration detected.

A total of five identical articles are analyzed and their hydrostatic head results recorded. Calculate and report the average hydrostatic head report to the nearest 0.1 mbar.

### EXAMPLE

Absorbent article Examples 1-2 and Comparative Example 1 were obtained as such.

Comparative Example 1: "Pampers 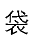 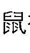 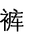 (Kangaroo Taped)" Size 4 purchased in 2022 in PRC.

Examples 1 and 2: Taped type absorbent article of Size 4 having an overall configuration similar to Comparative Example 1, and also having a composite isolation sheet as in Figures 1A, 2A, and 2B, with dimensions and longitudinal elastic elements as in Table 1 below. The longitudinal elastic bodies are positioned line symmetric about the longitudinal axis.

**Table 1**

| Dimension | Example 1 | Example 2 |
|---|---|---|
| Longitudinal dimension of the absorbent body L1 (mm) | 480 | 480 |
| Transverse dimension of the composite isolation sheet T1 (mm) | 140 | 140 |
| Distance between the side edges of the inner cuffs T2 (mm) | 75 | 75 |
| Longitudinal dimension of the back portion B1 (mm) | 75 | 75 |
| B1 / L1 (%) | 15.6 | 15.6 |
| Distance from back longitudinal end point to back end when back longitudinal elastic body fully stretched B2 (mm) | 90 | 90 |
| Distance from back longitudinal end point to back end when back longitudinal elastic body fully contracted B3 (mm) | 75 | 75 |
| B2 / B3 (%) | 83.3 | 83.3 |
| Number of longitudinal elastic strands / dtex | 2 / 620dtex | 4 / 620dtex |
| Distance of elastics from longitudinal axis (mm) | 1.5 | 7, 10 |

The articles of Example 1 and 2, when worn, provide the composite isolation sheet raised from the topsheet, while not covering the anal or urethral orifice of the intended wearer. The articles of Example 1 and 2 provide good isolation of body exudates from the wearer's skin, while maintaining the performance of containment and wear comfort of Comparative Example 1. The articles of Example 1 and 2 may be manufactured in approximately the same speed as manufacturing Comparative Example 1.

## Claims

1. An absorbent body (38) having a transverse direction, a longitudinal direction, a front end, a back end, a front region, a garment facing direction, and a wearer facing direction, comprising:
1) a water permeable topsheet (24);
2) a water impermeable backsheet (25);
3) an absorbent core (62) disposed between the topsheet and the backsheet;
4) a composite isolation sheet (CIS) bonded to the wearer facing side of the topsheet, the composite isolation sheet comprising:
a) a pair of inner cuffs (BLC) having a pair of side edges which are elasticized in the longitudinal direction;
b) a front portion (FC) in the front region having a front longitudinal end point (101) and a pair of front opening edges (102 ,103) wherein the pair of front opening edges are not elasticized wherein the front portion has a longitudinal dimension F1 of from about 8% to about 50% of the longitudinal dimension of the absorbent body; and
c) a front longitudinal elastic element (FE) disposed on the front portion, wherein the front longitudinal elastic element extends substantially in the longitudinal direction;
**characterized in that** the front longitudinal elastic element is an elastic body extending along the longitudinal axis (LX) between the front longitudinal end point towards the front end.

2. The absorbent body of Claim 1, wherein the distance from the front longitudinal end point to the front end when the front longitudinal elastic element is fully stretched is represented by F2, and the distance from the front longitudinal end point to the front end when the front longitudinal elastic element is fully contracted is represented by F3, wherein F3 is from about 30% to about 85% of F2.

3. An absorbent article (20) comprising the absorbent body of any of the preceding claims.

4. The absorbent article according to the preceding claim comprising application means, wherein the application means is selected from the group of a fastening means and an elastic belt.

## Patentansprüche

1. Absorptionskörper (38), der eine Querrichtung, eine Längsrichtung, ein vorderseitiges Ende, ein rückseitiges Ende, einen vorderseitigen Bereich, eine bekleidungsseitige Richtung und eine trägerseitige Richtung aufweist, umfassend:
1) eine wasserdurchlässige Oberschicht (24);
2) eine wasserundurchlässige Unterschicht (25);
3) einen Absorptionskern (62), der zwischen der Oberschicht und der Unterschicht angeordnet ist;
4) eine Verbundisolationslage (CIS), die mit der trägerseitigen Seite der Oberschicht gebunden ist, die Verbundisolationslage umfassend:
a) ein Paar von Innenbündchen (BLC), die ein Paar von Seitenrändern aufweisen, die in der Längsrichtung elastifiziert sind;
b) einen vorderseitigen Abschnitt (FC) in dem vorderseitigen Bereich, der einen vorderseitigen Längsendpunkt (101) und ein Paar von vorderseitigen Öffnungsrändern (102, 103) aufweist, wobei das Paar von vorderseitigen Öffnungsrändern nicht elastifiziert ist, wobei der vorderseitige Abschnitt eine Längsabmessung F1 von etwa 8 % bis etwa 50 % der Längsabmessung des Absorptionskörpers aufweist; und
c) ein vorderseitiges Längsgummibandelement (FE), das an dem vorderseitigen Abschnitt angeordnet ist, wobei sich das vorderseitige Längsgummibandelement im Wesentlichen in der Längsrichtung erstreckt;
**dadurch gekennzeichnet, dass** das vorderseitige Längsgummibandelement ein Gummibandkörper ist, der sich entlang der Längsachse (LX) zwischen dem vorderseitigen Längsendpunkt zu dem vorderseitigen Ende hin erstreckt.

2. Absorptionskörper nach Anspruch 1, wobei der Abstand von dem vorderseitigen Längsendpunkt zu dem vorderseitigen Ende, wenn das vorderseitige Längsgummibandelement vollständig gedehnt ist, durch F2 dargestellt wird und der Abstand von dem vorderseitigen Längsendpunkt zu dem vorderseitigen Ende, wenn das vorderseitige Längsgummibandelement vollständig kontrahiert ist, durch F3 dargestellt wird, wobei F3 von etwa 30 % bis etwa 85 % von F2 beträgt.

3. Absorptionsartikel (20), umfassend den Absorptionskörper nach einem der vorstehenden Ansprüche.

4. Absorptionsartikel nach dem vorstehenden Anspruch, umfassend ein Aufbringungsmittel, wobei das Aufbringungsmittel ausgewählt ist aus der Gruppe aus einem Verschlussmittel und einem Gummibandbund.

## Revendications

1. Corps absorbant (38) ayant une direction transversale, une direction longitudinale, une extrémité avant, une extrémité arrière, une région avant, une direction faisant face vers le vêtement, et une direction faisant face vers le porteur, comprenant :
1) une feuille de dessus perméables à l'eau (24) ;
2) une feuille de fond imperméable à l'eau (25) ;
3) une âme absorbante (62) disposée entre la feuille de dessus et la feuille de fond ;
4) une feuille d'isolation composite (CIS) liée au côté faisant face au porteur de la feuille de dessus, la feuille d'isolation composite comprenant :
a) une paire de revers intérieurs (BLC) ayant une paire de bords latéraux qui sont élastiques dans le sens longitudinal ;
b) une partie avant (FC) dans la région avant ayant un point d'extrémité longitudinal avant (101) et une paire de bords d'ouverture avant (102, 103), dans lequel la paire de bords d'ouverture avant n'est pas élastique, dans lequel la partie avant a une dimension longitudinale F1 allant d'environ 8 % à environ 50 % de la dimension longitudinale du corps absorbant ; et
c) un élément élastique longitudinal avant (FE) disposé sur la partie avant, dans lequel l'élément élastique longitudinal avant s'étend sensiblement dans la direction longitudinale ;
**caractérisé en ce que** l'élément élastique longitudinal avant est un corps élastique s'étendant le long de l'axe longitudinal (LX) entre le point d'extrémité longitudinale avant et l'extrémité avant.

2. Corps absorbant selon la revendication 1, dans lequel la distance du point d'extrémité longitudinale avant à l'extrémité avant lorsque l'élément élastique longitudinal avant est complètement étiré est représentée par F2, et la distance du point d'extrémité longitudinale avant à l'extrémité avant lorsque l'élément élastique longitudinal avant est complètement contracté est représentée par F3, dans lequel F3 représente d'environ 30 % à environ 85 % de F2.

3. Article absorbant (20) comprenant le corps absorbant selon l'une quelconque des revendications précédentes.

4. Article absorbant selon la revendication précédente, comprenant un moyen d'application, dans lequel le moyen d'application est choisi dans le groupe d'un moyen de fixation et d'une ceinture élastique.
